# FASCICULE DE BREVET EUROPEEN

(11) **EP 4 147 780 B1**
(45) Date de publication et mention de la délivrance du brevet: **04.10.2023**
(21) Numéro de dépôt: 22194262.6
(22) Date de dépôt: 07.09.2022
(51) Int. Cl.: B01L 3/00, C12M 3/06

(54) **COMPOSANT MICROFLUIDIQUE EMPLOYÉ POUR UNE MESURE D'IMPÉDANCE ÉLECTRIQUE À TRAVERS UN OBJET BIOLOGIQUE**
MIKROFLUIDISCHE KOMPONENTE, DIE ZUR MESSUNG DER ELEKTRISCHEN IMPEDANZ DURCH EIN BIOLOGISCHES OBJEKT VERWENDET WIRD
MICROFLUIDIC COMPONENT USED FOR A MEASUREMENT OF ELECTRICAL IMPEDANCE THROUGH A BIOLOGICAL OBJECT

(30) Priorité: 13.09.2021 FR 2109560
(43) Date de publication de la demande: 15.03.2023
(73) Titulaire: Commissariat à l'énergie atomique et aux énergies alternatives, 75015 Paris (FR)
(72) Inventeur: COSNIER, Marie-Line, 38054 GRENOBLE CEDEX 09 (FR); VERPLANCK, Nicolas, 38054 GRENOBLE CEDEX 09 (FR); ALESSIO, Manuel, 38054 GRENOBLE CEDEX 09 (FR); BUSSOOA, Anubhav, 38054 GRENOBLE CEDEX 09 (FR); MAILLEY, Pascal, 38054 GRENOBLE CEDEX 09 (FR); REVOL-CAVALIER, Frédéric, 38054 GRENOBLE CEDEX 09 (FR); ROUX, Jean-Maxime, 38054 GRENOBLE CEDEX 09 (FR)
(74) Mandataire: INNOV-GROUP

(56) Documents cités:
- US-A1- 2010 270 176
- US-A1- 2017 356 040
- US-B2- 8 454 813
- LUONGO KEVIN ET AL: "Microfluidic device for trapping and monitoring three dimensional multicell spheroids using electrical impedance spectroscopy", BIOMICROFLUIDICS , vol. 7, no. 3 5 juin 2013 (2013-06-05), pages 34108-34108, XP055911201, US ISSN: 1932-1058, DOI: 10.1063/1.4809590 Extrait de l'Internet: URL:https://www.ncbi.nlm.nih.gov/pmc/artic les/PMC3689825/pdf/BIOMGB-000007-034108_1. pdf [extrait le 2022-04-11]

## Description

### Domaine technique de l'invention

La présente invention se rapporte à un composant microfluidique employé pour une mesure d'impédance électrique à travers un objet biologique, ce composant pouvant être utilisé pour caractériser un objet biologique tel qu'un sphéroïde ou pour compter des objets biologiques présents dans un flux fluidique.

### Etat de la technique

Des études sont actuellement menées pour tenter de mieux caractériser un objet biologique tel qu'un sphéroïde formé par un amas de cellules biologiques. La caractérisation porte notamment sur la viabilité des cellules biologiques qui composent le sphéroïde. Des études montrent qu'il pourrait exister une corrélation entre la viabilité des cellules présentes dans le sphéroïde et des mesures d'impédance électrique à travers le sphéroïde. Autrement dit, plus l'impédance électrique mesurée à travers le sphéroïde serait forte, plus le sphéroïde contiendrait de cellules vivantes. Même si cette relation n'est pas encore complétement établie, de nombreuses études tendent à essayer de le démontrer. Ces études s'appuient sur l'utilisation de dispositifs microfluidiques de mesure d'impédance électrique. On peut notamment citer les études suivantes :
- Wu_2018 - Electrical impedance tomography for real-time and label-free cellular viability assays of 3D tumour spheroids - AnalystlUniversité d'Edinbourgh*.*
- Viswam_2018 - Impédance Spectroscopy and Electrophysiological Imaging of cells with a high-density CMOS microelectrode array system - IEEE Transactions on biomédical circuits and systems/Ecole polytechnique de Zurich (ETH)
- Heileman_2015 - Microfluidic platform for assessing pancreatic islet functionality through dieletric spectroscopy - Biomicrofluidics/Université McGill, Montréal

Les différents dispositifs utilisés dans ces études ne sont cependant pas satisfaisants pour les raisons suivantes :
- Les électrodes employées sont coplanaires et les objets biologiques à caractériser sont soit en contact avec les électrodes, soit trop éloignés. Dans certains cas, les lignes de champs produites entre les électrodes ne traverseront pas de façon optimale les objets biologiques à caractériser.
- Les matériaux utilisés pour l'intégration des électrodes dans ces systèmes fluidiques ne sont souvent pas transparents (matériaux d'électrodes ou de la chambre), ou la configuration des dispositifs n'est pas adaptée pour visualiser l'objet biologique et le surveiller. Il est souvent impossible de mettre en place un suivi par microscopie en transmission. Or ce mode d'observation est un standard en biologie.

Le brevet US8454813B2 décrit un dispositif de tri de cellules (cytomètre). Ce dispositif n'a pas pour objectif de caractériser un objet biologique par des mesures. Les électrodes sont en effet positionnées de manière à ce que le champ créé vienne plaquer la cellule contre le fond du puits.

La demande de brevet US2010/270176A1 décrit pour sa part un dispositif pour caractériser des neurones. Ce dispositif utilise des électrodes coplanaires agencées au fond de la cavité. Cette solution permet de venir plaquer l'objet biologique contre le fond de la cavité, ce qui n'est pas optimal pour le caractériser par des mesures.

Le but de l'invention est de proposer un composant microfluidique permettant de caractériser un objet biologique et qui soit :
- Facile à fabriquer ;
- Adapté pour réaliser des mesures d'impédance électrique fiables à travers l'objet biologique ;
- Adapté pour également effectuer un suivi optique, par exemple à l'aide d'un microscope en transmission ;

Le composant microfluidique doit présenter une structure dans laquelle l'objet biologique peut être surélevé par rapport au fond de la cavité et ainsi être traversé par les lignes de champ générées entre les deux électrodes.

### Exposé de l'invention

Ce but est atteint par un composant microfluidique employé pour une mesure d'impédance électrique à travers un objet biologique, ledit composant comprenant :
- Un espace microfluidique comprenant une zone dite de mesure,
- Au moins deux électrodes agencées en vis-à-vis, de part et d'autre de la zone de mesure,
- Le composant étant formé par l'assemblage d'au moins deux couches superposées suivant un plan de jonction longitudinal, dites couche inférieure et couche supérieure,
- Les deux couches présentant chacune au moins une cavité,
- Les deux couches étant assemblées l'une sur l'autre de manière à placer les deux cavités en vis-à-vis pour former ledit espace microfluidique,
- Les deux cavités ayant des sections transversales de tailles différentes, formant deux surfaces de dégagement de part et d'autre dudit espace microfluidique,
- Deux dépôts conducteurs électriques étant réalisés sur lesdites deux surfaces de dégagement de manière à former lesdites deux électrodes.

Selon une réalisation particulière, l'espace microfluidique comporte un piège hydrodynamique formant ladite zone de mesure.

Selon une autre réalisation particulière, la couche supérieure et/ou la couche inférieure présente une partie transparente, située en vis-à-vis de la zone de mesure.

Selon une autre réalisation particulière, l'espace microfluidique est réalisé sous la forme d'un canal creusé dans ledit composant microfluidique.

Selon une autre réalisation particulière, le piège hydrodynamique est réalisé sous la forme d'une marche agencée à l'intérieur dudit canal, en aval de la zone de mesure. Selon une autre réalisation particulière, le piège hydrodynamique est réalisé sous la forme d'un ou plusieurs piliers agencés à l'intérieur dudit canal, en aval de la zone de mesure.

Selon une autre réalisation particulière, l'espace microfluidique est réalisé sous la forme d'un puits creusé dans ledit composant microfluidique.

Selon une autre réalisation particulière, la couche inférieure et/ou la couche supérieure est réalisée dans un matériau choisi parmi le Cyclo-Olefin Copolymère, le Polyméthacrylate de Méthyle et un assemblage de silicium et de verre.

Selon une autre réalisation particulière, chaque dépôt conducteur électrique est réalisé dans un matériau métallique ou sous la forme d'une encre conductrice.

L'invention concerne un système de mesure d'impédance électrique à travers un objet biologique, comprenant un potentiostat comprenant deux bornes de connexion, ledit système comportant un composant microfluidique tel que défini ci-dessus, dont les deux électrodes sont connectées chacune à une borne distincte du potentisostat.

L'invention concerne un procédé de fabrication d'un composant microfluidique tel que défini ci-dessus, le procédé comporte des étapes de :
- Création d'une première cavité dans la couche inférieure,
- Dépôt d'une couche conductrice en au moins deux zones distinctes de la couche inférieure, de part et d'autre de la première cavité,
- Création d'une deuxième cavité dans la couche supérieure, ladite première cavité et ladite deuxième cavité étant créées avec des sections transversales distinctes afin de créer deux surfaces de dégagement occupées par la couche conductrice,
- Assemblage de la couche inférieure et de la couche supérieure, en plaçant la première cavité et la deuxième cavité en vis-à-vis de manière à créer ledit espace microfluidique, la couche conductrice étant agencée entre ladite couche inférieure et ladite couche supérieure.

Selon une particularité, le procédé comporte une étape de création d'un piège hydrodynamique dans l'espace microfluidique.

On constate que le composant de l'invention peut notamment être réalisé à l'aide d'un minimum de couches. Les deux électrodes sont surélevées par rapport au fond de la cavité, permettant aux lignes de champ de traverser l'objet biologique de part en part.

### Brève description des figures

D'autres caractéristiques et avantages vont apparaître dans la description détaillée qui suit faite en regard des dessins annexés dans lesquels :
- La figure 1 illustre, par une vue en coupe transversale, l'architecture du composant microfluidique de l'invention ;
- La figure 2 représente une vue de dessus du composant microfluidique de l'invention, selon une première variante de réalisation ;
- La figure 3 représente une vue de dessus du composant microfluidique de l'invention, selon une deuxième variante de réalisation ;
- La figure 4 illustre le principe de l'observation optique par transmission de l'objet biologique selon cette architecture ;
- La figure 5 illustre le principe de fabrication du composant microfluidique de l'invention ;
- La figure 6 illustre un premier principe de réalisation du piège hydrodynamique employé dans le composant microfluidique de l'invention ;
- La figure 7 illustre un deuxième principe de réalisation du piège hydrodynamique employé dans le composant microfluidique de l'invention ;
- La figure 8 représente un diagramme montrant la variation d'impédance mesurée à travers le milieu de culture, à travers un sphéroïde piégé dans la zone de mesure et à travers deux sphéroïdes piégés dans la zone de mesure ;

### Description détaillée d'au moins un mode de réalisation

Dans la suite de la description, les termes "inférieur", "supérieur", "au-dessus", "au-dessous" ou équivalent sont à considérer en tenant compte de la position du composant microfluidique sur un support horizontal.

Le terme "longitudinal" est à comprendre dans les directions parallèles au support horizontal et le terme "transversal" dans les directions perpendiculaires au support horizontal.

L'invention vise à permettre de mesurer l'impédance électrique à travers un objet biologique.

L'objet biologique est par exemple un agrégat de cellules. Par agrégat de cellules, on entend, selon l'invention, l'auto-assemblage d'un ou plusieurs types de cellules en trois dimensions. Un tel agrégat de cellules peut notamment s'appeler sphéroïde, organoïde, neuro-sphère. Cet agrégat peut également être un îlot de Langerhans. Dans la suite de la description, on utilisera de manière générique le terme "sphéroïde", référencé S, pour évoquer un tel agrégat, ce terme étant classiquement employé dans le domaine de la culture de cellules vivantes. De manière non limitative, un tel sphéroïde S peut par exemple présenter un diamètre allant de quelques dizaines de µm à quelques centaines de µm.

Le composant microfluidique 1 de l'invention peut se présenter sous la forme d'un support dans lequel est ménagé un espace microfluidique 10, de volume non nul, adapté à la présence d'un sphéroïde S. Cet espace microfluidique 10 peut se présenter sous la forme d'un canal, d'un puits ou équivalent.

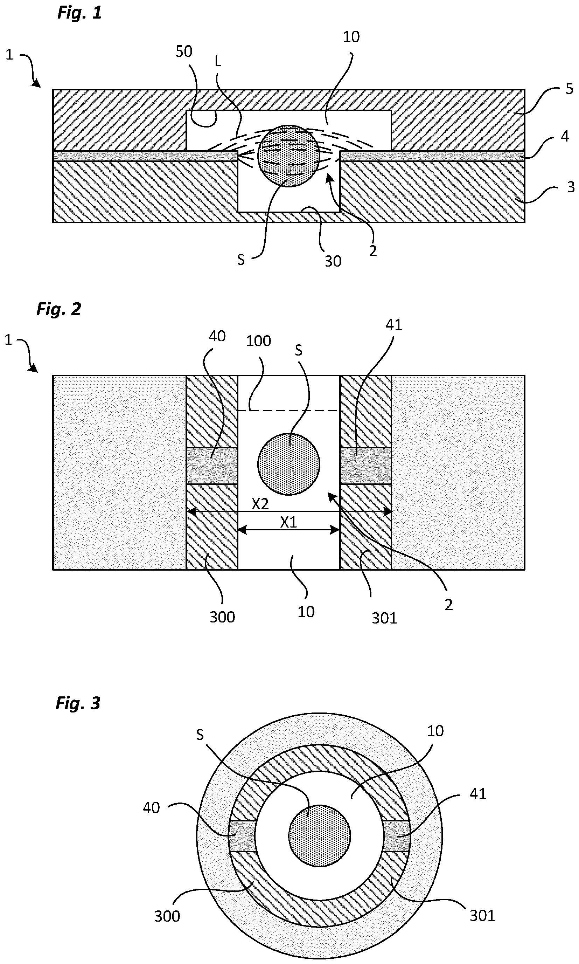

La figure 1 et la figure 2 montrent un espace microfluidique 10 réalisé sous la forme d'un canal. La figure 3 montre un espace microfluidique 10 réalisé sous la forme d'un puits. L'espace microfluidique peut être fermé au-dessus et au-dessous par l'assemblage des couches (voir ci-dessous).

L'espace microfluidique 10 comporte un piège hydrodynamique 100 agencé pour piéger le sphéroïde S et le maintenir dans une position stable définissant une zone de mesure 2 dans laquelle les mesures d'impédance peuvent être réalisées. On verra ci-après que ce piège hydrodynamique 100 peut être réalisé selon différentes variantes de réalisation, via une solution mécanique et/ou fluidique.

Comme représenté sur la figure 1, le composant microfluidique 1 est formé par l'assemblage de plusieurs couches 3, 4, 5 superposées. L'assemblage des couches permet de créer ledit espace microfluidique 10 ainsi que des électrodes 40, 41 nécessaires à la mesure des caractéristiques électriques du sphéroïde S. Cet espace microfluidique 10 est destiné à recevoir un liquide dans lequel est placé le sphéroïde S à caractériser électriquement. Le liquide est choisi avantageusement pour que le sphéroïde reste surélevé et ne vienne pas toucher le fond du piège. Il peut notamment présenter une composition ayant une viscosité suffisante pour maintenir le sphéroïde en hauteur et pour qu'il ne vienne pas toucher le fond du piège.

Le composant comporte ainsi :
- Une couche inférieure 3 dans laquelle est réalisée une première cavité 30 ;
- Une couche conductrice 4 déposée en au moins deux zones distinctes de la face supérieure de la couche inférieure 3, les deux zones étant séparées l'une de l'autre ;
- Une couche supérieure 5 dans laquelle est réalisée une deuxième cavité 50, de taille supérieure à la première cavité 30 ;

Lors de l'assemblage, la première cavité 30 et la deuxième cavité 50 sont mises en vis-à-vis de manière à former ledit espace microfluidique 10. Par cavité, on entend un trou non-traversant réalisé dans la couche considérée. Chaque couche (inférieure et supérieure) pourra être réalisée par la superposition de plusieurs couches/strates. En mettant les deux cavités en vis-à-vis, l'espace microfluidique 10 est fermé au-dessus et au-dessous.

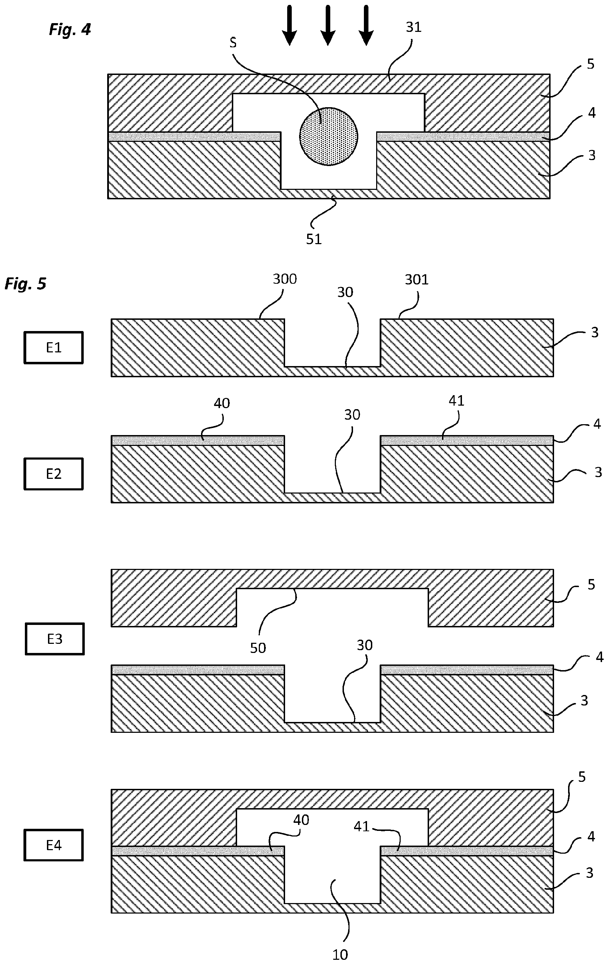

De manière avantageuse, comme représenté sur la figure 4, la couche inférieure 3 et/ou la couche supérieure 5 comportent chacune au moins une zone transparente 31, 51, situées l'une au-dessus de l'autre, dans l'axe de l'espace microfluidique pour créer une fenêtre de visualisation à travers la couche inférieure et la couche supérieure du support et permettre un suivi, par exemple par microscopie en transmission, à travers l'espace microfluidique 10.

La couche inférieure 3 et la couche supérieure 5 peuvent être réalisées dans un matériau transparent tel que COC (Cyclo-Olefin Copolymer), PMMA (Poly-méthacrylate de Méthyle) ou équivalent. La couche supérieure 5 peut également être fabriquée par assemblage de deux strates, une strate inférieure en silicium et une strate supérieure en verre. Bien entendu, d'autres variantes pourraient être envisagées.

Selon un aspect particulier de l'invention, les deux cavités 30, 50 ont des sections transversales distinctes l'une de l'autre, sur au moins une partie de leur longueur, de manière à créer deux surfaces de dégagement 300, 301, avantageusement coplanaires, situées dans le plan de jonction des deux couches de part et d'autre de la zone de mesure 2 de l'espace microfluidique 10. Sur les figures, la section transversale de la cavité 30 de la couche inférieure 3 est choisie inférieure à celle de la cavité 50 de la couche supérieure 5. Il faut noter que les sections transversales des deux cavités 30,50 ne sont pas forcément constantes sur toute la longueur du composant microfluidique 1. Elles sont juste distinctes au niveau de la zone de mesure pour créer les deux surfaces de dégagement 300, 301.

Chaque surface de dégagement 300, 301 comporte une zone de dépôt distincte de la couche conductrice 4 de manière à créer une électrode 40, 41 distincte sur chaque zone. En créant les deux surfaces de dégagement 300, 301, les électrodes se trouvent être en contact avec le liquide présent dans l'espace microfluidique, permettant de créer des lignes de champ L entre elles, traversant le sphéroïde S présent dans la zone de mesure 2.

A titre d'exemple et de manière non limitative, avec un espace microfluidique 10 réalisé sous la forme d'un canal, la cavité 30 réalisée dans la couche inférieure 3, formant une première partie de ce canal peut présenter une largeur X1 comprise entre 200 et 500µm et la cavité 50 réalisée dans la couche supérieure 5, pour former la deuxième partie du canal, peut présenter une largeur X2 comprise entre 300 et 700µm, sachant que l'on souhaite faire en sorte que X2 soit supérieur à X1, pour créer les deux surfaces de dégagement destinées à accueillir au moins partiellement les deux électrodes, de part et d'autre de la zone de mesure 2. Idéalement, les deux électrodes ont la même surface. Leur longueur est donc (X2-X1)/2. Les électrodes peuvent par exemple présenter une largeur comprise entre 100µm et 400µm.

Les électrodes 40, 41 sont préférentiellement métalliques et réalisées par évaporation après masquage, ou par technique de "lift-off". Les matériaux déposés peuvent dépendre de l'application (résistivité, biocompatibilité...) mais les matériaux utilisés classiquement sont l'or (avec sous-couche titane ou chrome) ou le platine. Une variante peut être l'utilisation d'encres conductrices déposées par exemple par sérigraphie, jet d'encre, spray.

Les deux électrodes 40, 41 sont ainsi séparées par la section de la cavité 30 définissant la zone de mesure 2, en amont du piège hydrodynamique 100 destiné à bloquer le sphéroïde S. Le sphéroïde S est amené dans la zone de mesure 2 pour subir les mesures d'impédance entre les deux électrodes.

Les deux électrodes 40, 41 peuvent occuper partiellement l'une des deux surfaces de dégagement créées autour de l'espace microfluidique 10 par la différence de section entre la cavité de la couche supérieure 5 et celle de la couche inférieure 3. Elles peuvent s'étendre dans la jonction entre les deux couches, couche inférieure 3 et couche supérieure 5 pour permettre une reprise de contact électrique déportée.

Selon un aspect particulièrement avantageux, les électrodes 40, 41 étant agencées dans un plan longitudinal, il est possible d'observer le sphéroïde S présent dans l'espace microfluidique 10, par transparence à travers la couche supérieure 5 et la couche inférieure 3 du support du composant 1.

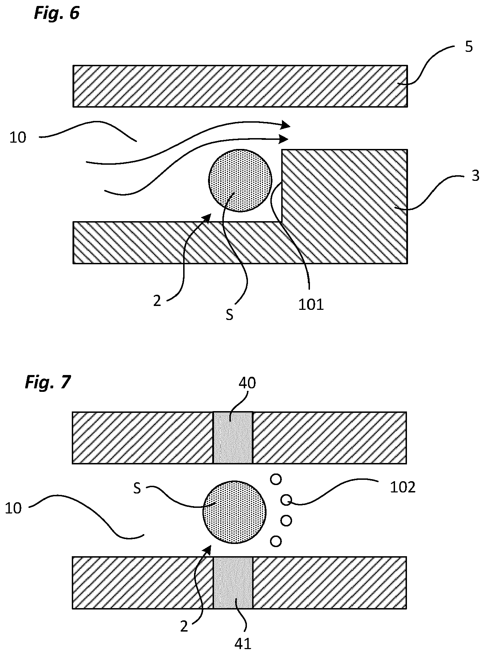

En référence aux figures 6 et 7, un piège hydrodynamique 100 est donc créé pour placer le sphéroïde S entre les deux électrodes 40, 41 dans l'espace microfluidique 10. Une première variante représentée sur la figure 6 consiste à créer une marche 101 dans l'espace microfluidique 10. A titre d'exemple, la figure 6 montre un espace microfluidique 10 réalisé sous la forme d'un canal. Une marche 101 est insérée à l'intérieur du canal, formant une butée pour le sphéroïde S lorsque celui-ci circule dans ledit canal.

La figure 7 montre un deuxième exemple de réalisation du piège hydrodynamique 100, formé par des piliers 102 agencés dans l'espace micro-fluidique 10 pour bloquer le sphéroïde S et le maintenir dans la zone de mesure.

Dans une autre variante de réalisation non représentée, le canal comporte un étranglement, situé en aval de la zone de mesure agencée entre les deux électrodes, cet étranglement formant le piège hydrodynamique 100. L'étranglement est suffisant pour bloquer le sphéroïde S et le maintenir dans la zone de mesure.

Dans une autre variante non représentée, le piège hydrodynamique 100 peut également être réalisé en employant un gel présent dans l'espace microfluidique 10, au niveau de la zone de mesure. Le sphéroïde S est injecté dans l'espace microfluidique 10 et maintenu en position dans la zone de mesure par gélification. Cette variante est également compatible avec les solutions de piégeage de type mécanique décrites ci-dessus.

Il faut noter que le composant microfluidique 1 de l'invention est associé à un potentiostat, aux bornes duquel chaque électrode 40, 41 du composant 1 vient se connecter.

L'assemblage entre les différentes couches 3, 4, 5 va dépendre des matériaux mais toutes les solutions pour assembler les matériaux cités ci-dessus sont envisageables, en particulier la thermocompression, la sérigraphie, la soudure laser, les adhésifs, les ultrasons voire les scellements direct et anodique pour des parties qui comportent du silicium et du verre.

Le composant microfluidique 1 peut notamment être employé pour compter des objets biologiques dans un flux. Pour cela, il s'agit de détecter les variations d'impédance électrique au niveau de la zone de mesure, chaque variation importante correspond au passage d'un objet biologique distinct.

La figure 5 illustre les différentes étapes du procédé de fabrication du composant microfluidique 1 de l'invention :
E1 : La couche inférieure 3 est préparée, afin d'y créer la cavité 30. Cette dernière peut être réalisée par micro-usinage, thermoformage, emboutissage à chaud, moulage par injection voire gravure chimique ou sèche dans le cas de silicium ou de verre, ou toute autre technique permettant de réaliser ce type de cavité. Les deux surfaces de dégagement 300, 301 sont présentes de part et d'autre de la cavité 30. Idéalement, la rugosité de ces surfaces est très faible (polie, voire qualité optique) pour optimiser l'adhésion de la couche 4 et l'assemblage avec la couche supérieure 5.
E2 : Sur la face supérieure de la couche inférieure 3, on dépose en deux zones distinctes, de part et d'autre de la cavité 30, une couche conductrice 4. Les deux électrodes formées occupent au moins partiellement les deux surfaces de dégagement 300, 301.
E3 : La couche supérieure 5 est préparée pour créer sa cavité 50. Cette cavité peut être réalisée avec les méthodes listées ci-dessus pour la cavité 30. Pour le même composant microfluidique 1, deux méthodes distinctes peuvent également être utilisées pour réaliser les cavités 30, 50. La cavité 50 est amenée contre la partie inférieure, formée de la couche inférieure 3 et de la couche conductrice 4. Les deux cavités sont placées en vis-à-vis de manière à créer l'espace microfluidique 10. Les deux électrodes 40, 41 sont présentes au moins partiellement sur les deux surfaces de dégagement 300, 301, créées par la différence de section entre les deux cavités 30, 50.
E4 : Le composant microfluidique 1 est assemblé et prêt à l'emploi.

Les différentes étapes de fabrication décrites ci-dessus sont adaptables à d'autres architectures de composant. Il faut également noter que le piège hydrodynamique 100 peut être créé lors de la première étape E1, pendant la création de la cavité 30 ou lors de la troisième étape E3, pendant la création de la cavité 50.

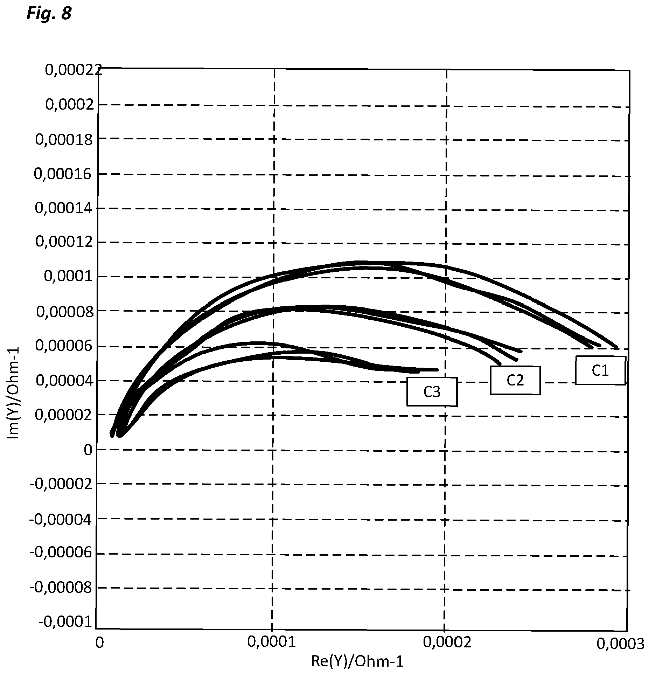

Le diagramme de la figure 8 représente l'impédance Im (par un diagramme de Nyquist) mesurée quand le canal fluidique est rempli de milieu de culture uniquement (courbes C1), quand un sphéroïde S est piégé par la marche agencée entre les électrodes (courbes C2) dans la zone de mesure 2 et quand deux sphéroïdes S sont piégés par la marche agencée entre les électrodes (courbes C3) dans la zone de mesure 2.

La solution de l'invention présente de nombreux avantages, parmi lesquels :
- Elle permet de mesurer l'impédance électrique à travers un objet biologique, de manière simple et fiable, tout en conservant une surveillance à travers les parties transparentes du support ;
- Elle est simple à fabriquer, par assemblage de couches, utilisant des techniques de fabrication classiques ;
- Elle est d'un coût peu élevé ;

## Revendications

1. Composant microfluidique (1) employé pour une mesure d'impédance électrique à travers un objet biologique, ledit composant comprenant :
- Un espace microfluidique (10) comprenant une zone dite de mesure (2),
- Au moins deux électrodes (40, 41) agencées en vis-à-vis, de part et d'autre de la zone de mesure (2),
- Le composant (1) étant formé par l'assemblage d'au moins deux couches superposées suivant un plan de jonction longitudinal, dites couche inférieure (3) et couche supérieure (5),
- Les deux couches présentant chacune au moins une cavité (30, 50),
- Les deux couches étant assemblées l'une sur l'autre de manière à placer les deux cavités en vis-à-vis pour former ledit espace microfluidique,
- **Caractérisé en ce que** :
- Les deux cavités (30, 50) ont des sections transversales de tailles différentes, formant deux surfaces de dégagement de part et d'autre dudit espace microfluidique (10),
- Deux dépôts conducteurs électriques sont réalisés sur lesdites deux surfaces de dégagement de manière à former lesdites deux électrodes (40, 41).

2. Composant microfluidique selon la revendication 1, **caractérisé en ce que** l'espace microfluidique (10) comporte un piège hydrodynamique (100) formant ladite zone de mesure (2).

3. Composant microfluidique selon la revendication 1 ou 2, **caractérisé en ce que** la couche supérieure (5) et/ou la couche inférieure (3) présente une partie transparente (31, 51), située en vis-à-vis de la zone de mesure.

4. Composant microfluidique selon l'une des revendications 1 à 3, **caractérisé en ce que** l'espace microfluidique (10) est réalisé sous la forme d'un canal creusé dans ledit composant microfluidique.

5. Composant microfluidique selon la revendication la revendication 4, **caractérisé en ce que** le piège hydrodynamique (100) est réalisé sous la forme d'une marche (101) agencée à l'intérieur dudit canal, en aval de la zone de mesure.

6. Composant microfluidique selon la revendication 4, **caractérisé en ce que** le piège hydrodynamique est réalisé sous la forme d'un ou plusieurs piliers (102) agencés à l'intérieur dudit canal, en aval de la zone de mesure (2).

7. Composant microfluidique selon l'une des revendications 1 à 3, **caractérisé en ce que** l'espace microfluidique (10) est réalisé sous la forme d'un puits creusé dans ledit composant microfluidique.

8. Composant microfluidique selon l'une des revendications 1 à 3, **caractérisé en ce que** la couche inférieure (3) et/ou la couche supérieure (5) est réalisée dans un matériau choisi parmi le Cyclo-Olefin Copolymère, le Polyméthacrylate de Méthyle et un assemblage de silicium et de verre.

9. Composant microfluidique selon l'une des revendications 1 à 8, **caractérisé en ce que** chaque dépôt conducteur électrique est réalisé dans un matériau métallique ou sous la forme d'une encre conductrice.

10. Système de mesure d'impédance électrique à travers un objet biologique, comprenant un potentiostat comprenant deux bornes de connexion, **caractérisé en ce qu'**il comporte un composant microfluidique tel que défini dans l'une des revendications 1 à 9, dont les deux électrodes (40, 41) sont connectées chacune à une borne distincte du potentisostat.

11. Procédé de fabrication d'un composant microfluidique (1) tel que défini dans l'une des revendications 1 à 9, **caractérisé en ce qu'**il comporte des étapes de :
- Création d'une première cavité (30) dans la couche inférieure,
- Dépôt d'une couche conductrice (4) en au moins deux zones distinctes de la couche inférieure, de part et d'autre de la première cavité (30),
- Création d'une deuxième cavité (50) dans la couche supérieure, ladite première cavité et ladite deuxième cavité étant créées avec des sections transversales distinctes afin de créer deux surfaces de dégagement (300, 301) occupées par la couche conductrice (4),
- Assemblage de la couche inférieure (3) et de la couche supérieure (5), en plaçant la première cavité (30) et la deuxième cavité (50) en vis-à-vis de manière à créer ledit espace microfluidique (10), la couche conductrice (4) étant agencée entre ladite couche inférieure (3) et ladite couche supérieure (5).

12. Procédé selon la revendication 11, **caractérisé en ce qu'**il comporte une étape de création d'un piège hydrodynamique (100) dans l'espace microfluidique (10).

## Patentansprüche

1. Mikrofluidische Komponente (1), die zur Messung der elektrischen Impedanz durch ein biologisches Objekt verwendet wird, wobei die Komponente umfasst:
- einen mikrofluidischen Raum (10), der einen Messbereich genannten Bereich (2) umfasst,
- mindestens zwei einander gegenüber angeordnete Elektroden (40, 41) beiderseits des Messbereichs (2),
- wobei die Komponente (1) durch Zusammenfügen von mindestens zwei entlang einer längs verlaufenden Verbindungsebene übereinanderliegenden Schichten, untere Schicht (3) und obere Schicht (5) genannt, gebildet wird,
- wobei die zwei Schichten jeweils mindestens eine Vertiefung (30, 50) aufweisen,
- wobei die zwei Schichten so aufeinander zusammengefügt sind, dass die zwei Vertiefungen gegenüberliegend angeordnet sind, um den mikrofluidischen Raum zu bilden,
- **dadurch gekennzeichnet, dass**:
- die zwei Vertiefungen (30, 50) Querschnitte unterschiedlicher Größen aufweisen, die zwei Freiflächen beiderseits des mikrofluidischen Raumes (10) bilden,
- zwei elektrisch leitende Ablagerungen auf den zwei Freiflächen ausgebildet sind, um so die zwei Elektroden (40, 41) zu bilden.

2. Mikrofluidische Komponente nach Anspruch 1, **dadurch gekennzeichnet, dass** der mikrofluidische Raum (10) eine hydrodynamische Falle (100) aufweist, die den Messbereich (2) bildet.

3. Mikrofluidische Komponente nach Anspruch 1 oder 2, **dadurch gekennzeichnet, dass** die obere Schicht (5) und/oder die untere Schicht (3) einen transparenten Teil (31, 51) aufweist, der sich gegenüber dem Messbereich befindet.

4. Mikrofluidische Komponente nach einem der Ansprüche 1 bis 3, **dadurch gekennzeichnet, dass** der mikrofluidische Raum (10) in der Form eines Kanals ausgebildet ist, der in der mikrofluidischen Komponente gebohrt ist.

5. Mikrofluidische Komponente nach Anspruch 4, **dadurch gekennzeichnet, dass** die hydrodynamische Falle (100) in der Form einer Stufe (101) ausgebildet ist, die im Inneren des Kanals, stromabwärts des Messbereichs, angeordnet ist.

6. Mikrofluidische Komponente nach Anspruch 4, **dadurch gekennzeichnet, dass** die hydrodynamische Falle in der Form einer oder mehrerer Säulen (102) ausgebildet ist, die im Inneren des Kanals, stromabwärts des Messbereichs (2), angeordnet sind.

7. Mikrofluidische Komponente nach einem der Ansprüche 1 bis 3, **dadurch gekennzeichnet, dass** der mikrofluidische Raum (10) in der Form eines Schachts ausgebildet ist, der in die mikrofluidische Komponente gebohrt ist.

8. Mikrofluidische Komponente nach einem der Ansprüche 1 bis 3, **dadurch gekennzeichnet, dass** die untere Schicht (3) und/oder die obere Schicht (5) aus einem Material ausgebildet ist, das aus Cycloolefin-Copolymer, Polymethylmethacrylat und einem Verbund von Silicium und Glas ausgewählt ist.

9. Mikrofluidische Komponente nach einem der Ansprüche 1 bis 8, **dadurch gekennzeichnet, dass** jede elektrisch leitende Ablagerung aus einem metallischen Material oder in der Form einer leitfähigen Tinte ausgebildet ist.

10. System zur Messung der elektrischen Impedanz durch ein biologisches Objekt, welches einen Potentiostaten umfasst, der zwei Anschlussklemmen umfasst, **dadurch gekennzeichnet, dass** es eine mikrofluidische Komponente aufweist, wie in einem der Ansprüche 1 bis 9 definiert, deren zwei Elektroden (40, 41) jeweils an eine andere Klemme des Potentiostaten angeschlossen sind.

11. Verfahren zur Herstellung einer mikrofluidischen Komponente (1), wie in einem der Ansprüche 1 bis 9 definiert, **dadurch gekennzeichnet, dass** es die Schritte umfasst:
- Erzeugung einer ersten Vertiefung (30) in der unteren Schicht,
- Ablagerung einer leitenden Schicht (4) in mindestens zwei verschiedenen Bereichen der unteren Schicht beiderseits der ersten Vertiefung (30),
- Erzeugung einer zweiten Vertiefung (50) in der oberen Schicht, wobei die erste Vertiefung und die zweite Vertiefung mit unterschiedlichen Querschnitten erzeugt werden, um zwei Freiflächen (300, 301) zu erzeugen, die von der leitenden Schicht (4) eingenommen werden,
- Zusammenfügen der unteren Schicht (3) und der oberen Schicht (5), wobei die erste Vertiefung (30) und die zweite Vertiefung (50) so gegenüberliegend angeordnet werden, dass der mikrofluidische Raum (10) erzeugt wird, wobei die leitende Schicht (4) zwischen der unteren Schicht (3) und der oberen Schicht (5) angeordnet wird.

12. Verfahren nach Anspruch 11, **dadurch gekennzeichnet, dass** es einen Schritt der Erzeugung einer hydrodynamischen Falle (100) in dem mikrofluidischen Raum (10) umfasst.

## Claims

1. Microfluidic component (1) used for measuring electrical impedance across a biological object, said component comprising:
- a microfluidic space (10) comprising a zone referred to as measurement zone (2),
- at least two electrodes (40, 41) arranged facing one another on each side of the measurement zone (2),
- the component (1) being formed by assembling, along a longitudinal junction plane, at least two superposed layers referred to as lower layer (3) and upper layer (5),
- the two layers each having at least one cavity (30, 50),
- the two layers being assembled with one another in such a way as to position the two cavities facing one another in order to form said microfluidic space,
- **characterized in that**:
- the two cavities (30, 50) have cross sections of different sizes, forming two clearance surfaces, one on each side of said microfluidic space (10),
- two electrically conducting deposits are applied to said two clearance surfaces so as to form said two electrodes (40, 41).

2. Microfluidic component according to Claim 1, **characterized in that** the microfluidic space (10) comprises a hydrodynamic trap (100) forming said measurement zone (2).

3. Microfluidic component according to Claim 1 or 2, **characterized in that** the upper layer (5) and/or the lower layer (3) has a transparent part (31, 51) situated facing the measurement zone.

4. Microfluidic component according to one of Claims 1 to 3, **characterized in that** the microfluidic space (10) is produced in the form of a canal hollowed into said microfluidic component.

5. Microfluidic component according to Claim 4, **characterized in that** the hydrodynamic trap (100) is produced in the form of a step (101) arranged inside said canal, downstream of the measurement zone.

6. Microfluidic component according to Claim 4, **characterized in that** the hydrodynamic trap is produced in the form of one or more posts (102) arranged inside said canal, downstream of the measurement zone (2).

7. Microfluidic component according to one of Claims 1 to 3, **characterized in that** the microfluidic space (10) is produced in the form of a well hollowed into said microfluidic component.

8. Microfluidic component according to one of Claims 1 to 3, **characterized in that** the lower layer (3) and/or the upper layer (5) is made from a material selected from cyclic olefin copolymer, polymethyl methacrylate, and an assembly of silicon and of glass.

9. Microfluidic component according to one of Claims 1 to 8, **characterized in that** each electrically conducting deposit is made from a metallic material or in the form of a conducting ink.

10. System for measuring electrical impedance across a biological object, comprising a potentiostat comprising two connection terminals, **characterized in that** said system comprises a microfluidic component as defined in one of Claims 1 to 9, of which the two electrodes (40, 41) are each connected to a distinct terminal of the potentiostat.

11. Method for manufacturing a microfluidic component (1) as defined in one of Claims 1 to 9, **characterized in that** said method comprises steps of:
- creating a first cavity (30) in the lower layer,
- depositing a conducting layer (4) in at least two distinct zones of the lower layer, on each side of the first cavity (30),
- creating a second cavity (50) in the upper layer, said first cavity and said second cavity being created with distinct cross sections so as to create two clearance surfaces (300, 301) which are occupied by the conducting layer (4),
- assembling the lower layer (3) and the upper layer (5) by placing the first cavity (30) and the second cavity (50) to face one another so as to create said microfluidic space (10), the conducting layer (4) being arranged between said lower layer (3) and said upper layer (5).

12. Method according to Claim 11, **characterized in that** said method comprises a step of creating a hydrodynamic trap (100) in the microfluidic space (10).
